(19) Europäisches Patentamt
European Patent Office
Office européen des brevets

(11) Veröffentlichungsnummer: **0 474 042 B1**

# (12) EUROPÄISCHE PATENTSCHRIFT

(45) Veröffentlichungstag der Patentschrift: **24.05.95**

(51) Int. Cl.[6]: **C07C 69/734**, C07D 213/55, C07D 237/16, C07D 239/54, C07D 241/24, C07D 333/24, C07D 277/30, C07D 307/54, C07D 233/60, C07D 249/06, C07D 261/08, //A01N37/38, A01N37/10,A01N43/10, A01N43/08,A01N43/40, A01N43/48,A01N43/647, A01N43/74,A01N43/80

(21) Anmeldenummer: **91114033.3**

(22) Anmeldetag: **22.08.91**

Die Akte enthält technische Angaben, die nach dem Eingang der Anmeldung eingereicht wurden und die nicht in dieser Patentschrift enthalten sind.

## (54) Alpha-Arylacrylsäurederivate, ihre Herstellung und Verwendung zur Bekämpfung von Schädlingen und Pilzen.

(30) Priorität: **07.09.90 DE 4028391**

(43) Veröffentlichungstag der Anmeldung:
**11.03.92 Patentblatt 92/11**

(45) Bekanntmachung des Hinweises auf die Patenterteilung:
**24.05.95 Patentblatt 95/21**

(84) Benannte Vertragsstaaten:
**BE CH DE DK ES FR GB IT LI NL**

(73) Patentinhaber: **BASF Aktiengesellschaft**
**Carl-Bosch-Strasse 38**
**D-67063 Ludwigshafen (DE)**

(72) Erfinder: **Kirstgen, Reinhard, Dr.**
**Erkenbrechtstrasse 23e**
**W-6730 Neustadt (DE)**
Erfinder: **Harreus, Albrecht, Dr.**
**Teichgasse 13**
**W-6700 Ludwigshafen (DE)**
Erfinder: **Kardorff, Uwe, Dr.**
**D 3, 4**
**W-6800 Mannheim 1 (DE)**
Erfinder: **Kuekenhoehner, Thomas, Dr.**
**Seidelstrasse 2**
**W-6710 Frankenthal (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 474 042 B1

(56) Entgegenhaltungen:
**EP-A- 0 178 826**
**EP-A- 0 203 606**
**EP-A- 0 229 974**
**EP-A- 0 256 667**
**EP-A- 0 278 595**
**EP-A- 0 280 185**
**EP-A- 0 335 519**

Erfinder: **Rang, Harald, Dr.**
**Maximilianstrasse 30**
**W-6700 Ludwigshafen (DE)**
Erfinder: **Lorenz, Gisela, Dr.**
**Erlenweg 13**
**W-6730 Neustadt (DE)**
Erfinder: **Ammermann, Eberhard, Dr.**
**Sachsenstrasse 3**
**W-6700 Ludwigshafen (DE)**
Erfinder: **Kuenast, Christoph, Dr.**
**Salierstrasse 2**
**W-6701 Otterstadt (DE)**

## Beschreibung

Die vorliegende Erfindung betrifft α-Arylacrylsäurederivate der allgemeinen Formel I,

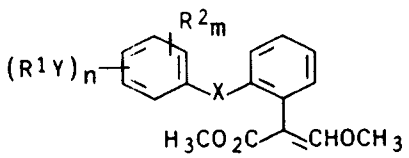

(I)

in der die Substituenten und Indizes folgende Bedeutung haben:

X

Ethylen oder Ethenylen;

Y

eine direkte Bindung oder Sauerstoff;

$R^1$

ein über ein C-Atom gebundenes ein- oder zweikerniges aromatisches System, welches neben Kohlenstoffgliedern ein bis vier Stickstoffatome oder ein oder zwei Stickstoffatome und/oder ein Sauerstoff- oder ein Schwefelatom enthalten kann, wobei dieses aromatische System ein bis sieben Halogenatome und/oder ein bis vier der folgenden Reste tragen kann: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_3$-$C_6$-Cycloalkyl, $C_5$-$C_6$-Cycloalkenyl, $C_2$-$C_4$-Alkenyl oder $C_2$-$C_4$-Alkinyl;

$R^2$

Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

n

1 oder 2, wobei die Reste verschieden sein können, wenn n 2 bedeutet;

m

0, 1 oder 2, wobei die Reste verschieden sein können, wenn m 2 bedeutet;

mit der Maßgabe, daß $R^1$ nicht Phenyl bedeutet, wenn n für 1 und m für 0 steht.

Außerdem betrifft die Erfindung Verfahren zur Herstellung dieser Verbindungen I, sie enthaltende Mittel sowie Verfahren zur Bekämpfung von Schädlingen mit Hilfe von α-Arylacrylsäurederivaten der allgemeinen Formel I',

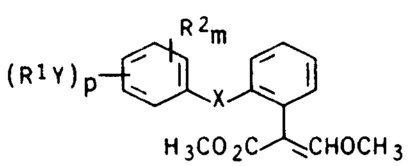

(I')

in der die Substituenten $R^1$, $R^2$, X und Y und der Index m die vorstehend gegebene Bedeutung haben und p für 1 oder 2 steht, mit der Maßgabe, daß $R^1$ auch Phenyl bedeuten kann, wenn X für Ethenylen, p für 1 und m für 0 steht sowie Verfahren zur Bekämpfung von Pilzen mit Hilfe von α-Arylacrylsäurederivaten der allgemeinen Formel I.

Aus der Literatur sind α-Arylacrylsäurederivate als Fungizide (EP-A 203 606, EP-A 229 974), als Insektizide und Fungizide (EP-A 178 826) und als Insektizide (EP-A 256 667, EP-A 335 519) bekannt.

Aufgabe der vorliegenden Erfindung waren neue wirksame Insektizide und Fungizide.

Demgemäß wurden die eingangs definierten α-Arylacrylsäurederivate I gefunden. Außerdem wurden Verfahren zur Herstellung dieser α-Arylacrylsäurederivate und sie enthaltende Mittel sowie Verfahren zu ihrer Verwendung gefunden.

Die α-Arylacrylsäurederivate I sind auf verschiedenen beispielsweise in der eingangs zitierten Literatur beschriebenen Wegen zugänglich. Besonders vorteilhaft erhält man sie nach einem der im nachfolgenden beschriebenen Verfahren A, B und C.

Verfahren A:

Man erhält die α-Arylacrylsäurederivate der Formel I in denen X Ethenylen bedeutet beispielsweise dadurch, daß man eine Phosphonsäure der allgemeinen Formel II, in an sich bekannter Weise (z.B. EP-A 203 606; J. Am. Chem. Soc. 83, 1732 (1961)) in einem inerten organischen Lösungsmittel in Gegenwart einer Base mit einem Benzaldehyd der allgemeinen Formel III umsetzt.

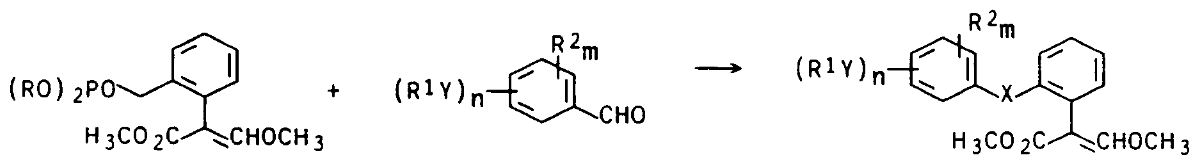

II III I

R in der Formel II steht dabei für eine $C_1$-$C_8$-Alkylgruppe wie insbesondere Methyl, Ethyl, Propyl und 1-Methylethyl.

Die Umsetzung erfolgt im allgemeinen bei Temperaturen von -30 bis 60 °C, vorzugsweise von 0 bis 40 °C.

Als Lösungsmittel eignen sich beispielsweise Diethylether, Benzol, Toluol, Tetrahydrofuran, Dimethoxyethan, Methanol, Ethanol und Dimethylformamid.

Insbesondere kommen Tetrahydrofuran und Diemthylformamid in Betracht.

Als Basen finden in diesem Verfahren n-Butyllithium, Natriumhydrid, Natriummethanolat, Kalium-t.-butylat, Natrium-t.-amylat, Lithiumdimethylamid und Lithiumbistrimethylsilylamid Verwendung.

Die Herstellung der benötigten Vorprodukte ist beispielsweise in der eingangs zitierten Literatur beschrieben.

Verfahren B:

Man erhält die α-Arylacrylsäurederivate der Formel I in denen X Ethenylen bedeutet beispielsweise auch dadurch, daß man eine Phosphonsäure der allgemeinen Formel IV in an sich bekannter Weise in einem inerten organischen Lösungsmittel in Gegenwart einer Base mit einem Benzaldehyd der Formel V umsetzt.

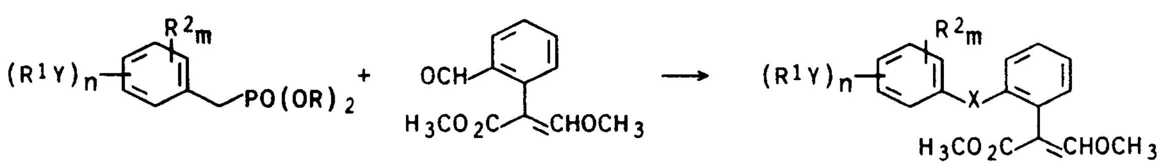

IV V I

R in der Formel IV steht dabei für eine $C_1$-$C_8$-Alkylgruppe wie insbesondere Methyl, Ethyl, Propyl und 1-Methylethyl.

Die Umsetzung erfolgt im allgemeinen bei Temperaturen von -30 bis 60 °C, vorzugsweise von 0 bis 40 °C.

Als Lösungsmittel und als Basen eignen sich im allgemeinen und im besonderen die bei Verfahren A genannten.

Die Herstellung der benötigten Vorprodukte ist beispielsweise in Houben-Weyl, Bd. XII/1, S. 433 ff (1963) beschrieben.

Verfahren C:

Man erhält die α-Arylacrylsäurederivate der Formel I in denen X Ethylen bedeutet beispielsweise dadurch, daß man eine Verbindung der allgemeinen Formel I, in der X Ethenylen bedeutet in an sich bekannter Weise (Houben-Weyl, Bd. V/2b, 264-267 (1981); Houben-Weyl, Bd. IV/1c, 580 ff. (1980); J. Am. Chem. Soc. 83, 4302 ff. (1961)) in einem inerten organischen Lösungsmittel reduziert.

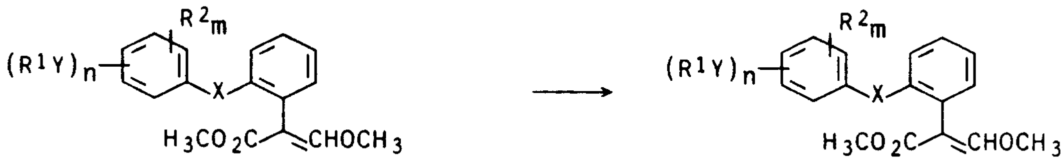

IA X = CH=CH

IB X = $CH_2CH_2$

Die Umsetzung erfolgt im allgemeinen bei Temperaturen von 0 bis 100 °C, vorzugsweise von 20 bis 40 °C.

Als Lösungsmittel eignen sich beispielsweise Essigsäure, Ethanol, Essigsäureethylester und Tetrahydrofuran oder entsprechende Gemische.

Als Reduktionsmittel eignen sich beispielsweise Wasserstoff in Gegenwart von Edelmetallkatalysatoren wie Pd, Pd/$CaCO_3$, Pd/C, Pt bei einem Wasserstoffdruck von 1 bis 10 bar oder Diimin, welches in situ aus Hydrazin und Sauerstoff in Gegenwart von Cu-II-Ionen erzeugt wird.

Im Hinblick auf die bestimmungsgemäße Verwendung der Verbindungen I in insektiziden und fungiziden Mitteln kommen als Substituenten und Indizes folgende Reste in Betracht:

X

Ethylen ($-CH_2-CH_2-$) oder Ethenylen ($-CH=CH-$);

Y

eine direkte Bindung oder Sauerstoff;

$R^1$

ein über ein C-Atom gebundenes ein- oder zweikerniges aromatisches System, welches neben Kohlenstoffgliedern ein bis vier Stickstoffatome oder ein oder zwei Stickstoffatome und ein Sauerstoff- oder ein Schwefelatom enthalten kann, wie Phenyl, Naphthyl, 2-Pyrryl, 3-Pyrryl, 3-Pyrazolyl, 4-Pyrazolyl, 5-Pyrazolyl, 2-Imidazolyl, 4-Imidazolyl, 5-Imidazolyl, 3-(1,2,4-Triazol)yl, 5-(1,2,4-Triazol)yl, 2-Pyridyl, 3-Pyridyl, 4-Pyridyl, 2-Pyrimidyl, 4-Pyrimidyl, 5-Pyrimidyl, 2-Pyrazinyl, 3-Pyrazinyl, 2-(1,3,5-Triazin)yl, 3-Indolyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-(1,3,4-Oxadiazol)yl, 2-(1,3,4-Thiadiazol)yl, 2-Benzoxazolyl, 2-Benzthiazolyl;

wobei dieses aromatische System ein bis sieben Halogenatome wie Fluor, Chlor, Brom und Jod, insbesondere Fluor und Chlor;

und/oder ein bis vier der folgenden Reste tragen kann: Cyano; Nitro;

$C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl, Ethyl, 1-Methylethyl und 1,1-Dimethylethyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, Ethoxy, 1-Methylethoxy und 1,1-Dimethylethoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy, 1,1,2,2-Tetrafluorethoxy und Pentafluorethyloxy, insbesondere Trifluormethyloxy, 1,1,2,2-Tetrafluorethoxy und Pentafluorethyloxy;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;

$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

$C_3$-$C_6$-Cycloalkyl wie Cyclopropyl, Cyclobutyl, Cyclopentyl und Cyclohexyl, vorzugsweise Cyclopropyl und Cyclopentyl;

$C_5$-$C_6$-Cycloalkenyl wie Cyclopent-1-enyl, Cyclopent-2-enyl, Cyclopent-3-enyl, Cyclohex-1-enyl, Cyclohex-2-enyl und Cyclohex-3-enyl, vorzugsweise Cyclopent-2-enyl und Cyclopent-3-enyl;
$C_2$-$C_4$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl, vorzugsweise Ethenyl und 1-Propenyl;
oder $C_2$-$C_4$-Alkinyl wie Ethinyl, 1-Propinyl, 2-Propinyl, 1-Butinyl, 2-Butinyl, 3-Butinyl und 1-Methyl-2-propinyl, vorzugsweise Ethinyl und 1-Propinyl;

$R^2$

Cyano, Nitro, Halogen,
$C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl, Ethyl, 1-Methylethyl und 1,1-Dimethylethyl;
$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, Ethoxy, 1-Methylethoxy und 1,1-Dimethylethoxy;
$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethyloxy und Pentafluorethyloxy;
$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;
$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

n

1 oder 2, wobei die Reste verschieden sein können, wenn n 2 bedeutet;

m

0, 1 oder 2, wobei die Reste verschieden sein können, wenn m 2 bedeutet,
mit der Maßgabe, daß $R^1$ nicht Phenyl bedeutet, wenn n für 1 und m für 0 steht.

Es versteht sich, daß die Verbindungen der Formel I in Abhängigkeit von den eingesetzten Ausgangsstoffen und den Umsetzungsbedingungen sowohl in Form von reinen Strukturisomeren, als auch in Form von Isomerenpaaren bzw. in Form von Isomerengemischen erhalten werden können und als solche als Wirkstoffe Verwendung finden können. Isomerengemische bzw. Isomerenpaare können in üblicher Weise in die sterisch einheitlichen Komponenten aufgetrennt werden. Die biologische Aktivität ist in Einzelfällen von der sterischen Konfiguration der Verbindungen abhängig.

Beispiele für insbesondere bevorzugte $\alpha$-Arylacrylsäurederivate der allgemeinen Formel IA bzw. IB sind in der folgenden Tabelle aufgeführt.

Tabelle

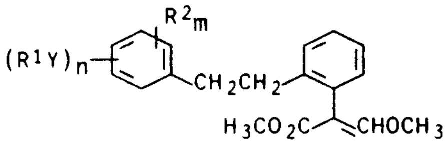

(IA)

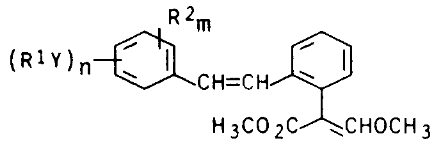

(IB)

| $(R^1Y)_n$ | $R^2_m$ |
|---|---|
| 3-Phenyl | 2-$CH_3$ |
| 3-Phenyl | 2-F |
| 3-Phenyl | 2-Cl |
| 3-Phenyl | 2-Br |
| 3-Phenyl | 2-$C_2H_5$ |
| 3-Phenyl | 2-$CH=CH_2$ |
| 3-Phenyl | 2-$CF_3$ |
| 3-Phenyl | 2-$OCH_3$ |
| 3-Phenyl | 2-CN |
| 3-Phenyl | 2-$NO_2$ |
| 3-Phenyl | 4-$CH_3$ |
| 3-Phenyl | 4-Cl |
| 3-Phenyl | 4-F |
| 3-Phenyl | 6-$CH_3$ |
| 3-Phenyl | 6-Cl |
| 3-Phenyl | 6-F |
| 3-Phenyl | 5-Cl |
| 3-Phenyl | 5-F |
| 3-(3-F-Phenyl) | 2-$CH_3$ |
| 3-(2-$CH_3$-Phenyl) | 2-$CH_3$ |
| 3-Phenyl | 2,4-$F_2$ |
| 3-Phenyl | 2,4-$Cl_2$ |
| 3-Phenyl | 2,4-$(CH_3)_2$ |
| 3-Phenyl | 2,6-$F_2$ |
| 3-(2-F-Phenyl) | H |
| 3-(2-Cl-Phenyl) | H |
| 3-(2-$CH_3$-Phenyl) | H |
| 3-(3-F-Phenyl) | H |
| 3-(3-Cl-Phenyl) | H |
| 3-(3-$CH_3$-Phenyl) | H |
| 3-(3-$NO_3$-Phenyl) | H |
| 3-(3-$CH_3O$-Phenyl) | H |
| 3-(3,5-$Cl_2$-Phenyl) | H |
| 3-(4-F-Phenyl) | H |

Tabelle (Fortsetzung)

| $(R^1Y)_n$ | $R^2_m$ |
|---|---|
| 3-(4-Cl-Phenyl) | H |
| 3-(4-$CH_3$-Phenyl) | H |
| 3-(4-CN-Phenyl) | H |
| 3-(3-CN-Phenyl) | H |
| 3-(4-$NO_2$-Phenyl) | H |
| 3-(4-MeO-Phenyl) | H |
| 4-(2-$CH_3$-Phenyl) | H |
| 4-(2-Cl-Phenyl) | H |
| 4-(3-Cl-Phenyl) | H |
| 4-(3-$CH_3$-Phenyl) | H |
| 4-(3-$CH_3O$-Phenyl) | H |
| 4-(3-F-Phenyl) | H |
| 4-(2,5-$(CH_3O)_2$-Phenyl) | H |
| 4-(2,5-$(CH_3O)_2$-Phenyl) | 3-Cl |
| 4-(2,5-$(CH_3O)_2$-Phenyl) | 3-Br |
| 4-(2,5-$(CH_3O)_2$-Phenyl) | 2,3,6-$Cl_3$ |
| 4-(4-$CH_3$-Phenyl) | H |
| 4-(4-$CH_3O$-Phenyl) | H |
| 4-(4-$C_2H_5$-Phenyl) | H |
| 4-(4-F-Phenyl) | H |
| 4-(4-Cl-Phenyl) | H |
| 4-(2,4-(Cl-Phenyl) | H |
| 4-(2,6-(Cl-4-$CH_3$-Phenyl) | 2,6-$Cl_2$ |
| 4-(3-CN-Phenyl) | H |
| 4-(4-CN-Phenyl) | H |
| 4-(3-$NO_2$-Phenyl) | H |
| 4-(4-$NO_2$-Phenyl) | H |
| 3-(4-Cl-Phenoxy) | H |
| 3-(3-Cl-Phenoxy) | H |
| 3-(4-$CF_3$-Phenoxy) | H |
| 3-(3-$CF_3$-Phenoxy) | H |
| 3-(4-$CH_3O$-Phenoxy) | H |
| 3-(3-$CH_3O$-Phenoxy) | H |
| 3-(4-$CH_3$-Phenoxy) | H |
| 3-(3-$CH_3$-Phenoxy) | H |
| 3-(4-t$C_4H_9$-Phenoxy) | H |
| 3-(3,4-($Cl_2$-Phenoxy) | H |
| 3-(3,5-($Cl_2$-Phenoxy) | H |

Tabelle (Fortsetzung)

| $(R^1Y)_n$ | $R^2{}_m$ |
|---|---|
| 3-(2,5-$Cl_2$, 3-$CF_3$-Phenoxy) | 2-Cl |
| 3-(2-Cl, 5-F, 3-$CF_3$-Phenoxy) | 2-Cl |
| 3-(2,5-$Cl_2$, 3-$CF_3$-Phenoxy) | 2-Br |
| 3-(2-Cl, 5-F, 3-$CF_3$-Phenoxy) | 2-Br |
| 3-(2,5-$Cl_2$, 3-$CF_3$-Phenoxy) | 2-CN |
| 3-(2-Cl, 5-F, 3-$CF_3$-Phenoxy) | 2-CN |
| 4-Phenoxy | H |
| 4-(4-$CH_3$-Phenoxy) | H |
| 4-(3-$CH_3$-Phenoxy) | H |
| 4-(4-$C_2H_5$-Phenoxy) | H |
| 4-(3-$C_2H_5$-Phenoxy) | H |
| 4-(4-t$C_4H_9$-Phenoxy) | H |
| 4-(3-t$C_4H_9$-Phenoxy) | H |
| 4-(4-$CF_3$-Phenoxy) | H |
| 4-(3-$CF_3$-Phenoxy) | H |
| 4-(4-Cl-Phenoxy) | H |
| 4-(3-Cl-Phenoxy) | H |
| 4-(4-Br-Phenoxy) | H |
| 4-(3-Br-Phenoxy) | H |
| 4-(4-F-Phenoxy) | H |
| 4-(3-F-Phenoxy) | H |
| 4-(4-$CH_3O$-Phenoxy) | H |
| 4-(3-$CH_3O$-Phenoxy) | H |
| 4-(4-Phenoxyphenoxy) | H |
| 4-(2,3-$Cl_2$-Phenoxy) | H |
| 4-(3,4-$Cl_2$-Phenoxy) | H |
| 4-(2,5-$Cl_2$-Phenoxy) | H |
| 4-(3,5-$Cl_2$-Phenoxy) | H |
| 4-(3,5-$Cl_2$-Phenoxy) | H |
| 4-(3-Cl, 4-F-Phenoxy) | H |
| 4-(2-Cl, 4-$CH_3$-Phenoxy) | H |
| 4-(4-t$C_4H_9$-Phenoxy) | H |
| 4-Phenoxy | 3-F |
| 4-(3-Cl-Phenoxy) | 3-Cl |
| 4-(3-Br-Phenoxy) | 3-Cl |
| 4-(Pyrid-2-yl) | H |
| 3-(Pyrid-2-yl) | H |
| 4-(Pyrid-3-yl) | H |

Tabelle (Fortsetzung)

| $(R^1Y)_n$ | $R^2_m$ |
|---|---|
| 3-(Pyrid-3-yl) | H |
| 4-(5-$CF_3$-Pyrid-2-yl) | H |
| 3-(5-$CF_3$-Pyrid-2-yl) | H |
| 4-(6-$CF_3$-Pyrid-2-yl) | H |
| 3-(6-$CF_3$-Pyrid-2-yl) | H |
| 4-(3-$CH_3$-Pyrid-2-yl) | H |
| 3-(3-$CH_3$-Pyrid-2-yl) | H |
| 4-(3-Cl, 5-$CF_3$-Pyrid-2-yl) | H |
| 3-(3-Cl, 5-$CF_3$-Pyrid-2-yl) | H |
| 4-(Pyridazin-3-yl) | H |
| 3-(Pyridazin-3-yl) | H |
| 4-(Pyrimid-2-yl) | H |
| 3-(Pyrimid-2-yl) | H |
| 4-(3-$CF_3$-Pyrid-2-yl) | H |
| 3-(3-$CF_3$-Pyrid-2-yl) | H |
| 4-(3,6-($CH_3$-Pyrazin-2-yl) | H |
| 3-(3,6-($CH_3$-Pyrazin-2-yl) | H |
| 4-(Pyrazin-2-yl) | H |
| 3-(Pyrazin-2-yl) | H |
| 4-(Pyrid-3-yl) | H |
| 3-(Pyrid-3-yl) | H |
| 4-(6-$CF_3$-Pyrid-2-yl) | H |
| 3-(6-$CF_3$-Pyrid-2-yl) | H |
| 4-(Thienyl-2-yl) | H |
| 3-(Thienyl-2-yl) | H |
| 4-(Thienyl-3-yl) | H |
| 3-(Thienyl-3-yl) | H |
| 4-(Thiazol-2-yl) | H |
| 3-(Thiazol-2-yl) | H |
| 4-(3-Cl-Thien-2-yl) | H |
| 3-(3-Cl-Thien-2-yl) | H |
| 4-(Furan-3-yl) | H |
| 3-(Furan-3-yl) | H |
| 4-(5-$CH_3$-Imidazol-4-yl) | H |
| 3-(5-$CH_3$-Imidazol-4-yl) | H |
| 4-(Triazol-2-yl) | H |
| 3-(Triazol-2-yl) | H |

Tabelle (Fortsetzung)

| $(R^1Y)_n$ | $R^2_m$ |
|---|---|
| 4-(3-$CH_3$-1,2,4-Thiadiazol-5-yl) | H |
| 3-(3-$CH_3$-1,2,4-Thiadiazol-5-yl) | H |
| 4-(Isoxazol-5-yl) | H |
| 3-(Isoxazol-5-yl) | H |
| 4-(5-$CH_3$-Isoxazol-3-yl) | H |
| 3-(5-$CH_3$-Isoxazol-3-yl) | H |
| 4-(5-$nC_3H_7$-Isoxazol-3-yl) | H |
| 3-(5-$nC_3H_7$-Isoxazol-3-yl) | H |
| 4-Imidazolyl | H |
| 3-Phenyl | H |

Die Verbindungen der Formel I' sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Otiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amglyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Verbindungen I eignen sich als Fungizide.

Sie zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,

Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wriksamen Menge der Wirkstoffe behandelt. Die Anwendung erfolgt vor oder nach der Infektion der Materialien, Pflanzen oder Samen durch die Pilze.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 2.005 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 2.013 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 2.014 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 2.019 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 2.016 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 2.015 mit 10 Gew.-Teilen N-Methyl-$\alpha$-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 1.002 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 1.005 werden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff für die Bekämpfung von Insekten beträgt unter Freilandbedingungen 0,01 bis 3, vorzugsweise 0,05 bis 1 kg/ha.

Die Aufwandmengen in fungiziden Mitteln liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paeci lomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Beispiele

2-($\beta$-Methoxy-$\alpha$-methoxycarbonylvinyl)-3'-(3-trifluormethyl)phenoxystilben

0,8 g Natriumhydrid werden in 20 ml abs. Tetrahydrofuran (THF) vorgelegt. Hierzu tropft man bei Raumtemperatur innerhalb von 30 min eine Mischung von 7,9 g 2-($\beta$-Methoxy-$\alpha$-methoxycarbonylvinyl)-benzylphosphonsäuredimethylester und 7,3 g 3-(3-Trifluorphenoxy)-benzaldehyd in 100 ml abs. THF.

Man läßt über Nacht rühren, gibt die Reaktionsmischung auf 200 ml Eiswasser und extrahiert 3 x mit je 100 ml tert.-Butylmethylether (MTBE). Die organische Phase wird 3 x mit je 100 ml Wasser und einmal mit 100 ml gesättigter NaCl-Lösung ausgeschüttelt. Nach dem Trocknen über $MgSO_4$ wird das Lösungsmittel abgezogen. Das Rohprodukt (12,8 g) wird mit Toluol als Eluent über eine Kieselgelsäule gereinigt. Man erhält 8,8 g eines gelben Öls (E/Z-Verhältnis 90:10). (Wirkstoffbeispiel 2.006).

$\alpha$-[2-(3'-[3-Trifluormethyl]phenoxy)-phenethylphenyl]-$\beta$-methoxyacrylsäuremethylester

2,7 g 2-($\beta$-Methoxy-$\alpha$-methoxycarbonylviny)-3'-(3-trifluormethyl)phenoxystilben werden bei Raumtemperatur in 100 ml THF in Gegenwart von 1 g Pd/C (10 %ig) bei 0,05 bar Wasserstoffüberdruck hydriert. Nach Aufnahme von ca. 0,2 l Wasserstoff innerhalb von 3 h wird abfiltriert und zur Trockene einrotiert. Man erhält 2,5 g eines hellgelben Öls. (Wirkstoffbeispiel 1.002).

2-($\beta$-Methoxy-$\alpha$-methoxycarbonylvinyl)-2'-methyl-3'-(3-fluorphenyl)-stilben

0,75 g Natriumhydrid werden in 100 ml abs. Tetrahydrofuran (THF) vorgelegt. Hierzu tropft man bei Raumtemperatur eine Mischung von 7,5 g 2-($\beta$-Methoxy-$\alpha$-methoxycarbonylvinyl)-benzylphosphonsäuredimethylester und 5,1 g 2-Methyl-3-(3'-fluorphenyl)-benzaldehyd in 100 ml abs. THF. Man läßt über Nacht rühren, gibt die Reaktionsmischung auf Eiswasser und extrahiert 3 x mit je 100 ml tert.-Butylmethylether (MTBE). Die organische Phase wird 2 x mit je 100 ml gesättigter NaCl-Lösung ausgeschüttelt, über $Na_2SO_4$ getrocknet und eingeengt. Das Rohprodukt wird mit Toluol/Essigester (9:1) as Eluent über eine Kieselgelsäule gereinigt. Man erhält 3,2 g eines Öls (E/Z-Verhältnis 80:20). (Wirkstoffbeispiel 2.004).

$\alpha$-[2-(2'-Methyl-3'-[3-fluorphenyl])-phenethylphenyl]-$\beta$-methoxyacrylsäuremethylester

2 g 2-([-Methoxy-$\alpha$-methoxycarbonylvinyl)-2'-methyl-3'-(3-fluorphenyl)-stilben werden bei Raumtemperatur in 100 ml THF in Gegenwart von 1 g Pd/C (10 %ig) bei 0,3 bar Wasserstoffüberdruck hydriert. Nach 3 h wird abfiltriert und zur Trockne einrotiert. Man erhält 1,25 g eines Feststoffs. (Wirkstoffbeispiel 1.001).

Tabelle 1

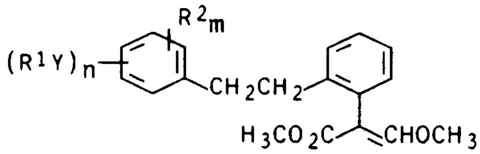

(IA)

| Nr. | $(R^1Y)_n$ | $R^2_m$ | Physik. Daten [Fp.(°C); NMR (ppm)] |
|---|---|---|---|
| 1.001 | 3-(3-F-Phenyl) | 2-$CH_3$ | 72 - 79 |
| 1.002 | 3-(3-$CF_3$-Phenoxy) | H | 7,5 (1H), 7,4-6,6 (11H), 3,7 (3H), 3,6 (3H), 2,7 (4H) |
| 1.003 | 3-(4-$CH_3$-Phenoxy) | H | 7,6 (1H), 7,3-6,7 (11H), 3,7 (3H), 3,6 (3H), 2,75 (4H), 2,3 (3H) |
| 1.004 | 3-(4-$C(CH_3)_3$-Phenoxy) | H | 7,5 (1H), 7,3-6,7 (11H), 3,7 (3H), 3,6 (3H), 2,7 (4H), 1,15 (9H) |
| 1.005 | 3-(3,5-$Cl_2$-Phenoxy) | H | 7,5 (1H), 7,2-6,6 (11H), 3,7 (3H), 3,6 (3H), 2,7 (4H) |

Tabelle 2

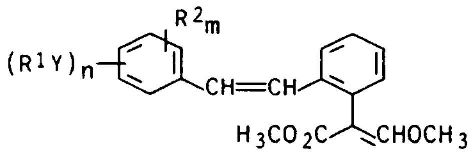

(IB)

| Nr. | $(R^1Y)_n$ | $R^2_m$ | Physik. Daten [Fp.(°C); NMR [ppm] |
|---|---|---|---|
| 2.001 | 3-Phenyl | $2-CH_3$ | 7,8 - 7,0 (14H); 6,3; 3,8 (3H); 3,7 (3H); 2,15 (3H) |
| 2.002 | 3-Phenyl | 2-F | 7,8 - 7,1 (15H); 3,8 (3H); 3,7 (3H) |
| 2.003 | 3-Phenyl | $2-CF_3$ | 7,8 - 7,1 (14H); 6,95 (1H); 3,8 (3H); 3,7 (3H) |
| 2.004 | 3-(3-F-Phenyl) | $2-CH_3$ | 7,8 - 6,9 (14H); 3,8 (3H); 3,7 (3H); 2,15 (3H) |
| 2.005 | 3-(4-Cl-Phenoxy) | H | 7,6 - 6,7 (15H); 3,7 (3H); 3,6 (3H) |
| 2.006 | 3-(3-$CF_3$-Phenoxy) | H | 7,7 - 6,8 (15H); 3,7 (3H); 3,6 (3H) |
| 2.007 | 3-(4-$CH_3O$-Phenoxy) | H | 7,65 - 6,7 (15H); 3,8 (3H); 3,7 (3H); 3,6 (3H) |
| 2.008 | 3-(4-$CH_3$-Phenoxy) | H | 7,8 - 6,8 (15H); 3,7 (3H); 3,6 (3H); 2,3 (3H) |
| 2.009 | 3-(4-t$C_4H_9$-Phenoxy) | H | 7,7 - 6,8 (15H); 3,7 (3H); 3,6 (3H); 1,3 (9H) |
| 2.010 | 3-(3,4-$Cl_2$-Phenoxy) | H | 7,6 - 6,7 (15H); 3,7 (3H); 3,6 (3H) |
| 2.011 | 3-(3,5-$Cl_2$-Phenoxy) | H | 7,6 - 6,7 (15H); 3,75 (3H); 3,65 (3H) |
| 2.012 | 4-Phenoxy | H | 7,75 (1H); 7,6 (1H); 7,5 - 6,9 (,14H); 3,8 (3H); 3,7 (3H) |
| 2.013 | 4-(3-$C_2H_5$-Phenoxy) | H | 7,7 (1H); 7,6 (1H); 7,45 - 6,8 (13H); 3,8 (3H); 3,65 (3H); 2,6 (2H); 1,2 (3H) |
| 2.014 | 4-(3-$CF_3$-Phenoxy) | H | 7,7 (1H); 7,6 (1H); 7,5 - 6,9 (13H); 3,8 (3H); 3,7 (3H) |

Tabelle 2 (Fortsetzung)

| Nr. | $(R^1Y)_n$ | $R^2_m$ | Physik. Daten [Fp.(°C); NMR [ppm] |
|---|---|---|---|
| 2.015 | 4-(3-Cl, 4-F-Phenoxy) | H | 7,75 (1H); 7,65 (1H); 7,45 (2H); 7,4 - 6,9 (10H); 3,8 (3H); 3,65 (3H) |
| 2.016 | 4-(Pyrid-3-yl) | H | 8,9 (1H); 8,6 (1H); 7,9 - 7,1 (13H); 3,75 (3H); 3,65 (3H) |
| 2.017 | 4-(Thienyl-3-yl) | H | 7,75 - 7,0 (14H); 3,8 (3H); 3,65 (3H) |
| 2.018 | 4-Imidazolyl | H | 7,9 - 7,0 (14H); 3,8 (3H); 3,65 (3H) |
| 2.019 | 3-Phenyl | H | 7,8 - 7,0 (16H); 3,8 (3H); 3,65 (3H) |

Anwendungsbeispiele

Die insektizide Wirkung der Verbindungen der allgemeinen Formel I' ließ sich durch folgende Versuche zeigen:

Die Wirkstoffe wurden

a) als 0,1 %ige Lösung in Aceton oder

b) als 10 %ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 bis 100 %ige Hemmung bzw. Mortalität hervorriefen (Wirkschwelle bzw. Minimalkonzentration).

A. Aphis fabae (Schwarze Laus), Kontaktwirkung

Stark befallene Buschbohnen (Vicia faba) wurden mit der wäßrigen Wirkstoffaufbereitung behandelt.

Nach 24 h wurde die Mortalitätsrate bestimmt.

In diesem Test zeigten die Verbindungen 2.013, 2.014, 2.015, 2.016 und 2.019 Wirkschwellen von 200 bis 1000 ppm.

B. Musca domestica (Stubenfliege), Zuchtversuch

25 ml einer trockenen Futtermischung (1 kg Kleie, 250 g Hefepulver, 35 g Fischmehl) wurde mit dem Wirkstoff und 25 ml einer Milchzuckerlösung (1 l Milch, 42 g Zucker) vermischt und anschließend mit 30 Larven des 1. Entwicklungsstadiums (L1) belegt.

Nach dem Schlüpfen der Larven in einem Kontrollexperiment ohne Wirkstoff wurde die Mortalität bestimmt.

In diesem Test zeigten die Verbindungen 2.005, 2.006, 2.013, 2.014, 2.015 und 2.016 Wirkschwellen von 10 bis 100 ppm.

C. Plutella maculipennis (Kohlschaben - Raupe), Kontaktwirkung

Blätter junger Kohlpflanzen wurden mit der wäßrigen Wirkstoffaufbereitung benetzt und anschließend auf einen angefeuchteten Filter gelegt. Die präparierten Blätter wurden anschließend mit jeweils 10 Raupen des 4. Entwicklungsstadiums belegt.

Nach 48 h wurde die Mortalitätsrate bestimmt.

In diesem Test zeigten die Verbindungen 2.002, 2.003, 2.005, 2.006, 2.009, 2.011, 2.012, 2.013, 2.014, 2.015, 2.016 und 2.019 Wirkschwellen von 100 bis 400 ppm.

D. Tetranychus telarius (Rote Spinne), Kontaktwirkung

Stark befallene getopfte Buschbohnen, die das zweite Folgeblattpaar zeigten, wurden mit wäßrigen Wirkstoffaufbereitung tropfnaß gespritzt.

Nach 5 Tagen im Gewächshaus wurde der Bekämpfungserfolg mittels Binokular bestimmt.

In diesem Test zeigten die Verbindungen 1.002, 1.003, 1.004, 1.005, 2.007, 2.008, 2.013 und 2.019 Wirkschwellen von kleiner als 200 bis 1000 ppm.

Die fungizide Wirkung der Verbindungen der allgemeinen Formel I ließ sich durch folgenden Versuch zeigen:

Die Wirkstoffe wurden als wäßrige Spritzbrühe aufbereitet, welche in der Trockensubstanz 80 % Wirkstoff und 20 % Emulgator enthielt. Als Vergleichsverbindung diente der aus der EP-A 203 606 als Beispiel Nr. 1 bekannte Wirkstoff A

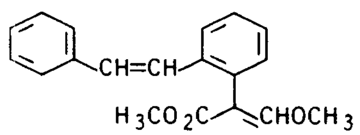

(A)

Puccinia recondita (Weizenbraunrost)

Mit Puccinia recondita Sporen bestäubte Weizensämlinge der Sorte "Frühgold" wurden 24 h bei 20 bis 22 °C und 90 bis 95 % Luftfeuchtigkeit incubiert. Die so vorbehandelten Pflanzen wurden mit der wäßrigen Wirkstoffaufbereitung behandelt.

Nach 8 Tagen im Gewächshaus wurde der Bekämpfungserfolg im Vergleich zu einer unbehandelten Kontrolle bestimmt.

In diesem Versuch wurde mit den Verbindungen 1.002, 1.003, 1.004, 1.005, 2.002, 2.005 und 2.008 eine erheblich bessere Bekämpfung erzielt als mit der Vergleichsverbindung A.

## Patentansprüche

**1.** $\alpha$-Arylacrylsäurederivate der allgemeinen Formel I,

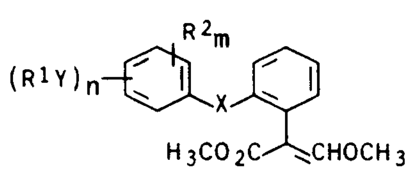

(I)

in der die Substituenten und Indizes folgende Bedeutung haben:

X

Ethylen oder Ethenylen;

Y

eine direkte Bindung oder Sauerstoff;

$R^1$

ein über ein C-Atom gebundenes ein- oder zweikerniges aromatisches System, welches neben Kohlenstoffgliedern ein bis vier Stickstoffatome oder ein oder zwei Stickstoffatome und/oder ein Sauerstoff- oder ein Schwefelatom enthalten kann, wobei dieses aromatische System ein bis sieben Halogenatome und/oder ein bis vier der folgenden Reste tragen kann: Cyano, Nitro, $C_1$-$C_4$-Alkyl, $C_1$-$C_4$-

Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_3$-$C_6$-Cycloalkyl, $C_5$-$C_6$-Cycloalkenyl, $C_2$-$C_4$-Alkenyl oder $C_2$-$C_4$-Alkinyl;
$R^2$
Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio; n
1 oder 2, wobei die Reste verschieden sein können, wenn n 2 bedeutet;
m
0, 1 oder 2, wobei die Reste verschieden sein können, wenn m 2 bedeutet;
mit der Maßgabe, daß $R^1$ nicht Phenyl bedeutet, wenn n für 1 und m für 0 steht.

**2.** Verfahren zur Herstellung der Verbindungen der Formel I gemäß Anspruch 1, in denen X Ethenylen bedeutet, dadurch gekennzeichnet, daß man eine Phosphonsäure der allgemeinen Formel II,

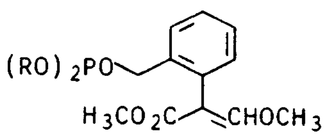

II

in der R für eine $C_1$-$C_8$-Alkylgruppe steht, in an sich bekannter Weise in einem inerten organischen Lösungsmittel in Gegenwart einer Base mit einem Benzaldehyd der allgemeinen Formel III,

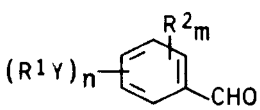

III

umsetzt.

**3.** Verfahren zur Herstellung der Verbindungen der Formel I gemäß Anspruch 1, in denen X Ethenylen bedeutet, dadurch gekennzeichnet, daß man eine Phosphonsäure der allgemeinen Formel IV,

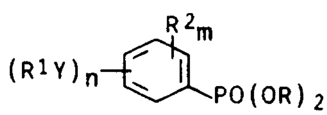

IV

in der R für eine $C_1$-$C_8$-Alkylgruppe steht, in an sich bekannter Weise in einem inerten organischen Lösungsmittel in Gegenwart einer Base mit einem Benzaldehyd der Formel V,

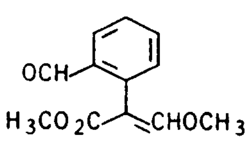

V

umsetzt.

**4.** Verfahren zur Herstellung der Verbindungen der Formel I gemäß Anspruch 1, in denen X Ethylen bedeutet, dadurch gekennzeichnet, daß man eine Verbindung der allgemeinen Formel I, in der X Ethenylen bedeutet in an sich bekannter Weise in einem inerten organischen Lösungsmittel reduziert.

**5.** Schädlingsbekämpfungsmittel und fungizide Mittel, enthaltend eine wirksame Menge eines α-Arylacrylsäurederivats der allgemeinen Formel I gemäß Anspruch 1 und inerte Zusatzstoffe.

**6.** Verfahren zur Bekämpfung von Schädlingen, dadurch gekennzeichnet, daß man die Schädlinge und/oder ihren Lebensraum mit einer wirksamen Menge eines α-Arylacrylsäurederivats der allgemeinen Formel I' behandelt,

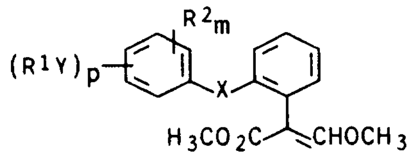

(I')

in der die Substituenten $R^1$, $R^2$, X und Y und der Index m die in Anspruch 1 gegebene Bedeutung haben und p für 1 oder 2 steht, mit der Maßgabe, daß $R^1$ auch Phenyl bedeuten kann, wenn X für Ethenylen, p für 1 und m für 0 steht.

**7.** Verfahren zur Bekämpfung von Pilzen, dadurch gekennzeichnet, daß man die Pilze und/oder ihren Lebensraum mit einer wirksamen Menge eines $\alpha$-Arylacrylsäurederivats der allgemeinen Formel I gemäß Anspruch 1 behandelt.

## Claims

**1.** An $\alpha$-arylacrylic acid derivative of the formula I

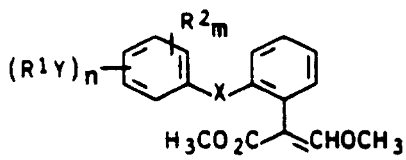

(I)

where X is ethylene or ethenylene;
Y is a direct bond or oxygen;
$R^1$ is a mononuclear or dinuclear aromatic system which is bonded via a carbon atom and, in addition to carbon members, may contain from one to four nitrogen atoms or one or two nitrogen atoms and/or an oxygen or a sulfur atom, and this aromatic system may carry from one to seven halogen atoms and/or from one to four of the following radicals: cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, $C_3$-$C_6$-cycloalkyl, $C_5$- or $C_6$-cycloalkenyl, $C_2$-$C_4$-alkenyl or $C_2$-$C_4$-alkynyl;
$R^2$ is cyano, nitro, halogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio;
n is 1 or 2, and the radicals may be different if n is 2, and
m is 0, 1 or 2, and the radicals may be different if m is 2,
with the proviso that $R^1$ is not phenyl when n is 1 and m is 0.

**2.** A process for the preparation of a compound of the formula I as claimed in claim 1, in which X is ethenylene, wherein a phosphonic acid of the formula II

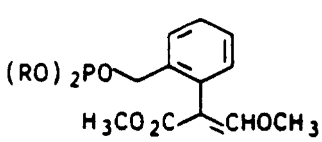

II

where R is $C_1$-$C_8$-alkyl, is reacted with a benzaldehyde of the formula III

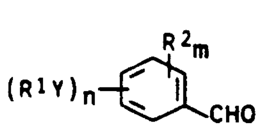

III

in a conventional manner in an inert organic solvent in the presence of a base.

3. A process for the preparation of a compound of the formula I as claimed in claim 1, in which X is ethenylene, wherein a phosphonic acid of the formula IV

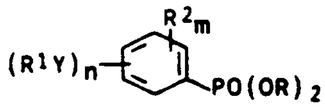

IV

where R is $C_1$-$C_8$-alkyl, is reacted with a benzaldehyde of the formula V

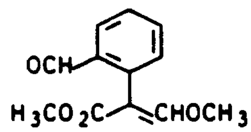

V

in a conventional manner in an inert organic solvent in the presence of a base.

4. A process for the preparation of a compound of the formula I as claimed in claim 1, in which X is ethylene, wherein a compound of the formula I in which X is ethenylene is reduced in a conventional manner in an inert organic solvent.

5. A pesticide or fungicide containing an effective amount of an $\alpha$-arylacrylic acid derivative of the formula I as claimed in claim 1 and inert additives.

6. A method for controlling pests, wherein the pests and/or their habitat are or is treated with an effective amount of an $\alpha$-arylacrylic acid derivative of the formula I'

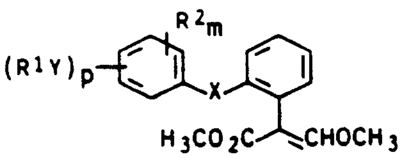

(I')

where $R^1$, $R^2$, X, Y and m have the meanings stated in claim 1 and p is 1 or 2, with the proviso that $R^1$ may also be phenyl when X is ethenylene, p is 1 and m is 0.

7. A method for controlling fungi, wherein the fungi and/or their habitat are or is treated with an effective amount of an $\alpha$-arylacrylic acid derivative of the formula I as claimed in claim 1.

## Revendications

1. Dérivés d'acide $\alpha$-arylacrilique de formule générale I

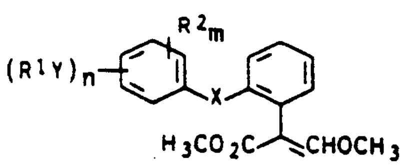

(I)

dans laquelle les substituants et les indices ont les significations suivantes:
X éthylène ou éthénylène ;
Y une liaison directe ou oxygène ;
$R^1$ un système aromatique à un ou deux cycles, lié par un atome de carbone, qui peut comporter, outre des chaînes de carbone, un à quatre atomes d'azote ou un ou deux atomes d'azote et/ou un atome d'oxygène ou de soufre, ce système aromatique pouvant porter un à sept atomes d'halogène

et/ou un à quatre des restes suivants :
cyano, nitro, alkyle en C1-C4, halogénoalkyle en C1-C4, alcoxy en C1-C4, halogénoalcoxy en C1-C4, alkylthio en C1-C4, halogénoalkylthio en C1-C4, cycloalkyle en C3-C6, cycloalcényle en C5-C6, alcényle en C2-C4 ou alcynyle en C2-C4 ;
$R^2$ cyano, nitro, halogène, alkyle en C1-C4, halogénoalkyle en C1-C4, alcoxy en C1-C4, halogénoalcoxy en C1-C4, alkylthio en C1-C4 ou halogénoalkylthio ;
n 1 ou 2, les restes pouvant être différents, lorsque n = 2 ;
m 0, 1 ou 2, les restes pouvant être différents, lorsque m = 2 ;
sous réserve que $R^1$ ne représente pas phényle lorsque n = 1 et m = 0.

**2.** Procédé de préparation des composés de formule I selon la revendication 1, dans lequel X représente éthénylène caractérisé par le fait que l'on fait réagir un acide phosphonique de formule générale II

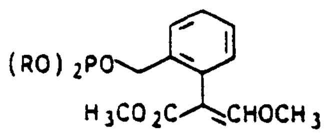

II

dans laquelle R représente un groupe alkyle en C1-C8, de manière connue dans un solvant organique inerte en présence d'une base, avec un benzaldéhyde de formule générale III

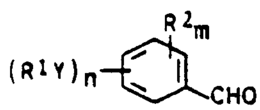

III

**3.** Procédé de préparation des composés de formule I selon la revendication 1, dans lequel X représente éthénylène caractérisé par le fait que l'on fait réagir un acide phosphonique de formule générale IV

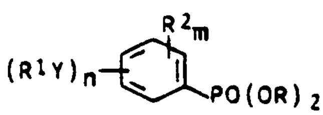

IV

dans laquelle R représente un groupe alkyle en C1-C8, de manière connue dans un solvant organique inerte en présence d'une base, avec un benzaldéhyde de formule générale V

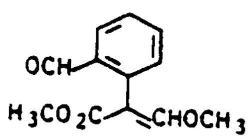

V

**4.** Procédé de préparation des composés de formule I selon la revendication 1, dans lequel X représente éthénylène caractérisé par le fait que l'on réduit un composé de formule générale I, dans laquelle X représente éthénylène, de manière en soi connue, dans un solvant organique inerte.

**5.** Agent pesticide et agent fongicide, comportant une quantité efficace d'un dérivé d'acide $\alpha$-arylacrylique de formule générale I selon la revendication 1 et des additifs inertes.

**6.** Procédé de lutte contre les parasites, caractérisé par le fait qu'on traite les parasites et/ou leur biotope avec une quantité efficace d'un dérivé d'acide $\alpha$-arylacrylique de formule générale I'

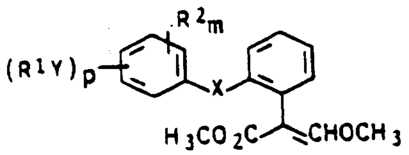

(I')

dans laquelle les substituants $R^1$, $R^2$, X et Y et l'indice m ont la signification donnée dans la revendication 1 et p vaut 1 ou 2 sous réserve que $R^1$ peut représenter aussi phényle, quand X est mis pour éthénylène, p pour 1 et m pour 0.

**7.** Procédé pour lutter contre les champignons, caractérisé par le fait que l'on traite les champignons ou leur biotope avec une quantité efficace d'un dérivé d'acide α-arylacrylique de formule générale I selon la revendication 1.